# EUROPÄISCHE PATENTSCHRIFT

(11) **EP 3 599 090 B1**
(45) Veröffentlichungstag und Bekanntmachung des Hinweises auf die Patenterteilung: **23.03.2022**
(21) Anmeldenummer: 19186607.8
(22) Anmeldetag: 16.07.2019
(51) Int. Cl.: B32B 7/12, B32B 15/08, B32B 15/085, B32B 15/088, B32B 15/09, B32B 15/20, B32B 27/08, B32B 27/10, B32B 27/30, B32B 27/32, B32B 27/34, B32B 27/36, B32B 29/00

(54) **FOLIENVERBUND ZUR VERPACKUNG TRANSDERMALER PFLASTER UND VERPACKUNG AUS EINEM SOLCHEN FOLIENVERBUND**
FILM COMPOSITE FOR PACKAGING TRANSDERMAL PATCHES AND PACKAGING FROM SUCH A FILM COMPOSITE
FILM COMPOSITE DESTINÉ À EMBALLER UN PANSEMENT TRANSDERMIQUE ET EMBALLAGE D'UN TEL FILM COMPOSITE

(30) Priorität: 24.07.2018 DE 102018212303; 11.10.2018 DE 102018125195
(43) Veröffentlichungstag der Anmeldung: 29.01.2020
(73) Patentinhaber: Huhtamaki Flexible Packaging Germany GmbH & Co. KG, 87671 Ronsberg (DE)
(72) Erfinder: HERB, Manuella, 87657 Görisried (DE); DORN, Michael, 87463 Dietmannsried (DE); STROH, Thomas, 87700 Memmingen (DE)
(74) Vertreter: Ruttensperger Lachnit Trossin Gomoll

(56) Entgegenhaltungen:
- WO-A1-2017/100182
- US-A1- 2015 225 151

## Beschreibung

Die vorliegende Erfindung betrifft einen Folienverbund zur Herstellung einer Verpackung für flächige Träger von pharmazeutischen Wirkstoffen, wie beispielsweise transdermale Pflaster, umfassend in Richtung von der designierten Außenseite des Folienverbunds zu seiner designierten Innenseite eine Metalllage, eine Acrylsäure-haltige Verbindungslage, eine Polyethylenlage und eine COC-Lage aus Cycloolefin-Copolymer, wobei eine von der Metalllage abgewandte Seite der COC-Lage eine freiliegende Fläche des Folienverbunds ist.

Ein solcher Folienverbund zu dem genannten Zweck einer Herstellung einer Verpackung von pharmazeutischen Wirkstoffträgern ist aus der WO 2015/123211 A1 bekannt. Der gattungsgemäße Folienverbund kommt dabei in unmittelbaren Kontakt mit dem flächigen Träger eines pharmazeutischen Wirkstoffs und damit mit dem pharmazeutischen Wirkstoff selbst. Es handelt sich bei den infrage kommenden pharmazeutischen Wirkstoffen um Wirkstoffe, wie etwa Nikotin, Fentanyl oder Lidocain. Die von diesen pharmazeutischen Wirkstoffen ausgehende Belastung des Folienverbundes ist erheblich. Es ist dabei sicherzustellen, dass zum einen der Folienverbund als solcher über längere Zeit erhalten bleibt, um eine unerwünschte Freisetzung des pharmazeutischen Wirkstoffs des Wirkstoffträgers zu verhindern. Selbst wenn der Folienverbund erhalten bleibt, also beispielsweise nicht unter dem Einfluss des in Verbundlagen diffundierenden pharmazeutischen Wirkstoffs wenigstens abschnittsweise delaminiert, muss zum anderen sichergestellt werden, dass der pharmazeutische Wirkstoff nicht durch den Folienverbund hindurch diffundieren bzw. migrieren kann.

Zur Erhöhung der Diffusions-Barrierewirkung des Folienverbunds gegenüber den durch sie verpackten pharmazeutischen Wirkstoffen ist es bekannt, den Anteil an Cycloolefin-Copolymer (COC) in der in der späteren Verpackung ganz innen liegenden COC-Lage möglichst hoch zu wählen. Mit der Erhöhung des COC-Gehalts in der COC-Lage wird es jedoch zunehmend schwieriger, die Stabilität des Folienverbunds aufrecht zu erhalten, etwa eine Delamination oder ähnliche Zerfallserscheinungen zu verhindern.

Auch die WO 2017/114922 A1 offenbart Folienverbünde zur Bildung von Verpackungen für flächige pharmazeutische Wirkstoffträger.

Es ist daher Aufgabe der vorliegenden Erfindung, den eingangs genannten Folienverbund mit größtmöglicher chemischer und mechanischer Stabilität gegenüber den zu verpackenden pharmazeutischen Wirkstoffen im Hinblick auf seine Aufgabe einer Bildung einer sicheren, auch kindersicheren Verpackung von flächigen pharmazeutischen Wirkstoffträgern auszubilden.

Diese Aufgabe wird erfindungsgemäß gelöst durch einen Folienverbund der eingangs genannten Art, bei welchem die Polyethylenlage Metallocen-Polyethylen umfasst. Versuche haben nämlich gezeigt, dass bei ansonsten identischen Dimensionen des Folienverbunds die Haftung der COC-Lage an der Polyethylenlage dann erheblich besser ist, wenn die Polyethylenlage Metallocen-Polyethylen C8 aufweist. "Metallocen-Polyethylen" ist dabei Polyethylen, welches unter Verwendung von Metallocen-Katalysatoren in an sich bekannter Weise hergestellt wurde.

Dabei ist der durch die Verwendung von Metallocen-Polyethylen gegenüber herkömmlichem Polyethylen erzielbare Haftungsgewinn in der Haftungswirkung zwischen der COC-Lage und der Polyethylenlage umso größer, je höher der COC-Gehalt in der COC-Lage ist. Da die COC-Lage zur Erzielung einer möglichst hohen Barrierewirkung gegenüber pharmazeutischen Wirkstoffen, die im flächigen Wirkstoffträger gespeichert sind, einen möglichst großen COC-Anteil von wenigstens 95 Gew-%, bevorzugt von wenigstens 99,8 Gew.-% an der Masse der gesamten COC-Lage aufweist, kann mit der vorliegend vorgestellten Verwendung von Metallocen-Polyethylen in der zwischen der Acrylsäule-haltigen Verbindungslage und der COC-Lage angeordneten Polyethylenlage ein besonders großer Haftungsvorteil erzielt werden.

Im Zweifel, etwa wenn mehr als eine Polymerlage des Folienverbunds COC enthält, soll eine COC-Lage nur dann als COC-Lage im Sinne der vorliegenden Anmeldung gelten, wenn sie bezogen auf ihre Lagenmasse mindestens 50 Gew.-% COC enthält. Die genannte Polyethylenlage, die Metallocen-Polyethylen enthält, ist vorzugsweise frei von COC.

Grundsätzlich kann bereits mit vergleichsweise geringen Mengen an Metallocen-Polyethylen eine Verbesserung der Haftung der COC-Lage an der Polyethylenlage erreicht werden. Besonders stark ist die Verbesserung der Haftungswirkung bei ansonsten abmessungsgleichen Vergleichsproben unterschiedlicher Folienverbundflecke dann, wenn der Anteil an Metallocen-Polyethylen am gesamten Polyethylen der Polyethylenlage wenigstens 50 Gew.-% umfasst. Grundsätzlich können also auch sogenannte Polyethylen-Blends zur Bildung der Polyethylenlage verwendet werden, in welchen Metallocen-Polyethylen mit Nicht-Metallocen-Polyethylen vermischt ist.

Wegen der vorteilhaften Haftung erhöhenden Wirkung von Metallocen-Polyethylen ist vorzugsweise dessen Anteil (stets in Gewichtsprozent) am gesamten in der Polyethylenlage enthaltenen Polyethylen möglichst groß, vorzugsweise größer als 90 Gew.-%, besonders bevorzugt größer als 96 Gew.-%.

Dabei hat es sich zur Erhöhung der Haftungswirkung und ebenso der Verarbeitbarkeit des Folienverbunds auf entsprechenden Verarbeitungsmaschinen zu daraus gebildeten Verpackungen als vorteilhaft erwiesen, wenn die Polyethylenlage ein Metallocen-LLDPE umfasst. Vorzugsweise umfasst die Polyethylenlage nur Metallocen-LLDPE als das Metallocen-Polyethylen.

Die Dichte des Metallocen-Polyethylens liegt zur Erzielung guter Laufeigenschaften einer Folienverbundbahn in den oben genannten Verarbeitungsmaschinen vorteilhafterweise in einem Bereich zwischen 0,917 und 0,921 g/m². Zusätzlich oder alternativ weist das Metallocen-Polyethylen zur Erzielung hochfester und hochdichter dauerhafter Siegelnähte einen Schmelzflussindex (MFR) von zwischen 6 und 10 g/10 min. auf, gemessen nach ISO 1133 bei einer Prüftemperatur von 190 °C und bei Verwendung eines Prüfkörpers mit einem Gewicht von 2,16 kg. Denn die Polyethylenlage kann zusätzlich zur COC-Lage als der außen liegenden Barriere- und Siegellage zu einer Siegelverbindung des Folienverbunds beitragen.

Jede Art von Metallocen-Polyethylen in der Polyethylenlage sorgt für eine gewünschte Erhöhung der Haftungswirkung der COC-Lage an der Polyethylenlage, verglichen mit einer Polyethylenlage gleicher Abmessung oder/und gleichen Flächengewichts in dem Folienverbund. Darüber hinaus kann jedoch durch Verwendung unterschiedlicher Typen von Metallocen-Polyethylen die Steifigkeit der Polyethylenlage und damit des Folienverbunds insgesamt beeinflusst werden. Eine vorteilhafte Steifigkeit der Polyethylenlage, bei vorgegebenen Abmessungen derselben, wird erfindungsgemäß dadurch erhalten, dass die Polyethylenlage ein Metallocen-Polyethylen C8 umfasst oder aus der genannten Metallocen-Polyethylen-Type besteht. Metallocen-Polyethylen C8 liefert nach bisherigen Versuchen bei vorgegebenem Folienverbund und insbesondere bei vorgegebener Abmessung oder/und Flächengewicht der Polyethylenlage das beste Ergebnis hinsichtlich der erzielten Lagensteifigkeit der Polyethylenlage einerseits und der erzielten Verbundsteifigkeit des Folienverbunds insgesamt andererseits.

Aus diesem Grunde ist es nachvollziehbarerweise vorteilhaft, wenn die Polyethylenlage als Metallocen-Polyethylen nur ein Metallocen-Polyethylen C8 umfasst. Gemäß den obigen Darlegungen ist dies besonders bevorzugt ein Metallocen-LLDPE C8.

Die Polyethylenlage ist bevorzugt im Extrusionsverfahren aufgetragen, besonders bevorzugt coextrudiert mit der COC-Lage.

Ein kommerziell verfügbares Metallocen-Polyethylen, welches hervorragend als Polyethylenlage in dem genannten Folienverbund einsetzbar ist, ist das von Dow Chemical Company unter dem Handelsnamen "Elite^{™} 5811" erhältliche Polyethylen.

Zur Erzielung einer möglichst großen Haftungswirkung des Teilverbunds aus Polyethylenlage und COC-Lage an der Metalllage umfasst die Acrylsäure-haltige Verbindungslage bevorzugt ein Ethylen-Acrylsäure-Copolymer (EAA-Copolymer) oder besteht aus diesem.

Bevorzugt wird auch die Acrylsäure-haltige Verbindungslage durch Extrusion auf die Metalllage aufgetragen. Besonders bevorzugt wird ein Teilverbund aus Acrylsäure-haltiger Verbindungslage, insbesondere EAA-Lage, Polyethylenlage und COC-Lage durch Coextrusion in einem gemeinsamen Extrusionsschritt auf die Metalllage aufgetragen.

Die Metalllage dient als Sauerstoff- und Gasbarriere zum Schutz eines Verpackungsinhalts vor äußeren Einflüssen und überdies als Barriere für pharmazeutische Wirkstoffe und deren Bestandteile, welche durch die COC-Lage, die Polyethylenlage und die Acrylsäure-haltige Verbindungslage hindurch aufgrund von Diffusion die Metalllage erreichen. Bevorzugt ist die Metalllage eine Aluminiumlage.

Bevorzugt weist jede der Lagen aus Acrylsäure-haltiger Verbindungslage, Polyethylenlage und COC-Lage eine Lagendicke im Bereich von etwa 7 µm bis 15µm auf. Dies sorgt zum einen für eine hervorragende Anbindung an die Metalllage. Zum anderen kann in diesem Dickenbereich nicht nur die COC-Lage, sondern auch die Polyethylenlage zur Herstellung einer Siegelverbindung mit der COC-Lage eines gleichartig aufgebauten Folienverbunds dienen. Da in dem genannten Fall auch die unter der COC-Lage gelegenen Polyethylenlage besonders gut und sicher zur Herstellung einer Siegelverbindung beitragen kann, sind die oben angegebenen Werte des Schmelzflussindex (MFR) zur Erzielung hervorragender Siegeleigenschaften des Folienverbunds mit der freiliegenden COC-Lage von großem Vorteil. Vorzugsweise liegen die Dickenwerte der drei, vorzugsweise coextrudierten, Lagen: Acrylsäure-haltige Verbindungslage, Polyethylenlage und COC-Lage, jeweils im Bereich von 9 µm bis 12 µm. Zur Erzielung hervorragender Siegeleigenschaften unter Beteiligung nicht nur der COC-Lage als außenliegende Siegellage, sondern auch der darunterliegenden Polyethylenlage unterscheiden sich vorzugsweise die Lagendicken der Polyethylenlage und der COC-Lage kaum. Auch die Dicke der Acrylsäure-haltigen Verbindungslage unterscheidet sich vorzugsweise kaum von der Dicke der beiden erstgenannten Lagen: Polyethylenlage und COC-Lage, um eine möglichst gute Anbindung der COC-Lage und der Polyethylenlage an die Metalllage zu erzielen, ohne dabei den Folienverbund unerwünscht dick zu gestalten. Bevorzugt unterscheiden sich die Dickenwerte der genannten Lagen - bezogen auf die dünnste der genannten Lagen - um nicht mehr als 15 %, vorzugsweise um nicht mehr als 11 %. Dann lässt sich ein Dreierverbund aus Acrylsäure-haltiger Verbindungslage, Polyethylenlage und COC-Lage gut coextrudieren, wobei der so entstehende Folienverbund sehr gute Laufeigenschaften auf Verarbeitungsmaschinen zur Verarbeitung des Folienverbunds zu einer Verpackung aufweist und überdies sehr gute Siegeleigenschaften aufweist.

Grundsätzlich kann der Folienverbund so verwendet werden, wie er oben beschrieben ist. Zum weiteren Schutz des Folienverbunds kann dieser auf der von der COC-Lage abgewandten Seite der Metalllage wenigstens eine weitere Lage aus Papier oder/und Kunststoff aufweisen. Dadurch ist die Metalllage eine vollständig innenliegende Lage des Folienverbunds und als solche besser vor äußeren Einflüssen geschützt. Da in der Regel die COC-Lage eine ganz innen liegende Lage einer aus dem Folienverbund gebildeten Verpackung ist, wird durch das Vorsehen der wenigstens einen weiteren Lage die Aufbringung von Produktinformationen auf der zum Verbraucher weisenden Außenseite des Folienverbunds erleichtert, die ansonsten mit erhöhtem Aufwand auf der Metalllage aufgebracht werden müssten.

Die wenigstens eine weitere Lage ist vorzugsweise auf die Metalllage oder auf eine auf dieser bereits vorhandene weitere Lage aufkaschiert. Hierzu wird bevorzugt ein lösungsmittelhaltiger Kaschierkleber mit einem Flächenauftragsgewicht von 2 bis 4 g/m², vorzugsweise mit einem Flächenauftragsgewicht von 3 g/m² verwendet.

Zur leichten Bedruckung und zur Kindersicherung einer aus dem Folienverbund gebildeten Verpackung vor unerwünschtem Öffnen und Freilegen der darin verpackten pharmazeutischen Wirkstoffe weist der Folienverbund bevorzugt auf der von der COC-Lage abgewandten Seite der Metalllage sowohl eine weitere Lage aus Papier als auch eine weitere Lage aus Kunststoff auf. Wegen der besonders einfachen Bedruckbarkeit der Papierlage und damit einer besonders einfachen und kostengünstigen Möglichkeit, den Folienverbund oder/und eine damit gebildete Verpackung mit Verbraucherinformationen zu versehen, ist bevorzugt die Kunststofflage näher bei der Metalllage gelegen ist als die Papierlage. Bevorzugt ist die Papierlage eine außen liegende Lage des Folienverbunds. Ein auf die Papierlage aufgedruckter Druckauftrag kann durch eine Schutzschicht, wie etwa einen Klarlack, wenigstens teilweise oder bevorzugt vollständig abgedeckt sein.

Zur Sicherung gegen unerwünschtes Aufreißen durch Kinder ist die weitere Lage aus Kunststoff bevorzugt ein biaxial orientierter Kunststoff.

Als Kunststoff für die wenigstens eine weitere Kunststofflage haben sich Polyethylenterephthalat oder/und Polyamid in Versuchen bewährt. Kunststofflagen aus den genannten Kunststoffen sind mechanisch und thermisch ausreichend stabil und stellen besonders in orientierter Form eine weitere Barrierewirkung gegen Diffusion von Gasen und Dämpfen bereit. Die weitere Kunststofflage kann somit in einer Richtung die Metalllage vor chemischen Angriffen von außen schützen und kann in der entgegengesetzten Richtung zusätzlich den pharmazeutischen Wirkstoff oder Bestandteile davon zurückhalten, falls diese die Metalllage überwinden sollten.

Dann, wenn der Folienverbund auf der von der COC-Lage wegweisenden Seite der Metalllage eine weitere Lage aus Papier aufweist, hat diese vorzugsweise eine Dicke von 35 bis 45 µm, besonders bevorzugt von 39 bis 41 µm. Zusätzlich oder alternativ kann die weitere Lage aus Papier ein Flächengewicht von 35 bis 45 g/m², insbesondere von 39 bis 41 g/m² aufweisen.

Dann, wenn der Folienverbund auf der von der COC-Lage wegweisenden Seite der Metalllage eine weitere Lage aus Kunststoff aufweist, hat diese vorzugsweise eine Dicke von 19 bis 27 µm, insbesondere von 22 bis 24 µm. Zusätzlich oder alternativ weist die zusätzliche Lage aus Kunststoff ein Flächengewicht von 28 bis 36 g/m², insbesondere von 31 bis 33 g/m² auf. Diese Werte von Dicken und Flächengewicht bieten einen sehr guten Kompromiss zwischen ausreichender mechanischer Festigkeit, thermischer Stabilität und geringer Gesamtdicke des Folienverbunds.

Da der oben beschriebene Folienverbund besonders geeignet ist zur Herstellung einer Verpackung für einen flächigen Träger von pharmazeutischen Wirkstoffen, wie beispielsweise ein transdermales Pflaster, betrifft die vorliegende Erfindung auch eine solche Verpackung, umfassend zwei Folienverbundflecke aus je einem Folienverbund, wie er oben beschrieben und weitergebildet ist. Die Folienverbundflecke sind unter Bildung eines Träger-Aufnahmeraums mit aufeinander zuweisenden COC-Lagen angeordnet und durch abschnittsweises Siegeln von COC-Lagenbereichen miteinander verbunden. Abschnitte der aufeinander zuweisenden COC-Lagenbereiche sind dabei miteinander gesiegelt, bevorzugt unter Beteiligung der unter den COC-Lagen gelegenen jeweiligen Polyethylenlage mit Metallocen-Polyethylen. Die Siegelnaht umfasst dann bevorzugt eine Mischung aus COC und aus Metallocen-Polyethylen. Die Siegelnaht erstreckt sich dann in Dickenrichtung bis in die Polyethylenlage hinein. Andere Abschnitte der aufeinander zuweisenden COC-Lagenbereiche der beiden Folienverbundflecke liegen einander unverbunden gegenüber. Dann, wenn in der Verpackung ein flächiger Träger von pharmazeutischen Wirkstoffen aufgenommen ist, befindet sich dieser zwischen einander unverbunden gegenüberliegenden Abschnitten je einer COC-Lage eines an der Bildung der Verpackung beteiligten Folienverbundflecks.

Als flächiger Träger von pharmazeutischen Wirkstoffen gilt ein Substrat, welches in seinen beiden zueinander und zur Substrat-Dickenrichtung orthogonalen Haupterstreckungsrichtungen jeweils Abmessungen aufweist, die um wenigstens das 10-Fache, vorzugsweise um wenigstens das 30-Fache, größer sind als die Abmessung des Substrats in Dickenrichtung und welches zur Abgabe eines im Substrat gespeicherten pharmazeutischen Wirkstoffs ausgebildet ist.

Die Verpackung kann raumsparend bei gleichzeitig dauerhaft hoher Abschirmung des darin verpackten pharmazeutischen Wirkstoffs ein Vier-Rand-Siegelbeutel mit um den Träger-Aufnahmeraum umlaufender Siegelnaht sein.

Ein wie oben beschrieben im Träger-Aufnahmeraum der Verpackung aufgenommenen flächiger Wirkstoffträger ist bevorzugt ein transdermales Pflaster. Der pharmazeutische Wirkstoff im Wirkstoffträger umfasst bevorzugt Nikotin oder/und Fentanyl oder/und Lidocain oder/und einen anderen zur Verabreichung mittels transdermalem Pflaster bekannten Wirkstoff.

Als "designierte" Innen- bzw. Außenseite des Folienverbunds sind jene Seiten des Folienverbunds bezeichnet, die an einer späteren aus dem Folienverbund gebildeten Verpackung einen Abschnitt der Verpackungsinnenseite bzw. der Verpackungsaußenseite bildet.

Bevorzugt weist der Folienverbund nur die in der vorliegenden Anmeldung genannten Lagen auf.

Die vorliegende Erfindung wird nachfolgend anhand der beiliegenden Zeichnungen näher erläutert werden. Es stellt dar:
- Figur 1: eine Querschnittsansicht durch eine erfindungsgemäße Ausführungsform eines Folienverbunds der vorliegenden Anmeldung,
- Figur 2: eine Draufsicht auf eine unter Beteiligung des Folienverbunds von Figur 1 gebildete Verpackung eines transdermalen Pflasters und
- Figur 3: eine Querschnittsansicht der Verpackung von Figur 2 entlang der Schnittebene III-III von Figur 2.

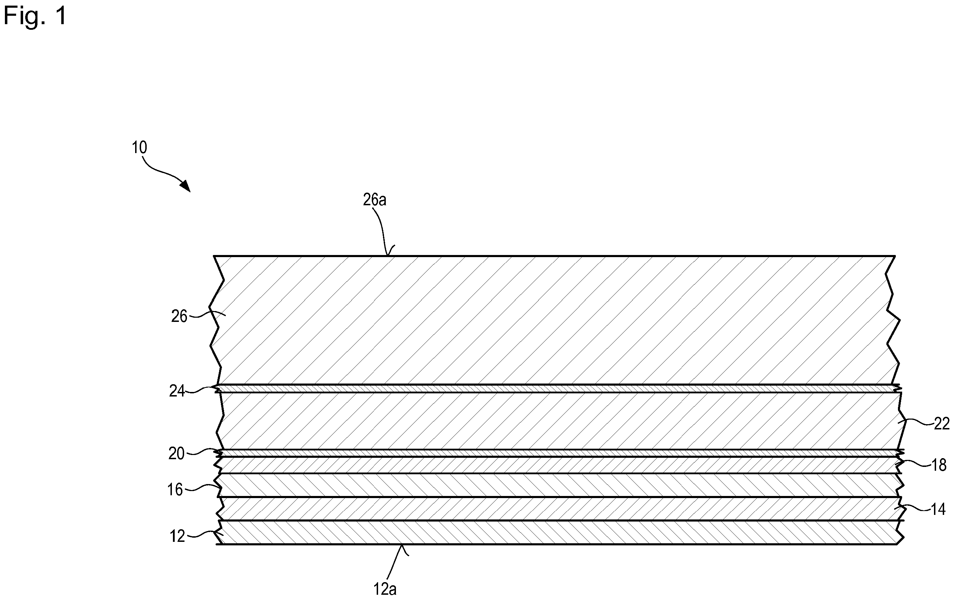

In Figur 1 ist eine erfindungsgemäße Ausführungsform eines Folienverbunds der vorliegenden Anmeldung allgemein mit 10 bezeichnet. Die Darstellung der Figur 1 ist lediglich schematisch. Insbesondere die Dicken der einzelnen Lagen des Folienverbunds 10 sind nicht maßstabsgerecht abgebildet. Der Folienverbund 10 weist auf seiner designierten Innenseite eine COC-Lage 12 auf, welche einen COC-Gehalt von etwa 99,8 Gew.-% aufweist. Der Anteil an COC in der COC-Lage 12 ist möglichst hoch gewählt, um mit der COC-Lage 12 eine möglichst gute Barrierelage gegen Migration und Diffusion von pharmazeutischen Wirkstoffen, wie Nikotin oder/und Fentanyl oder/und Lidocain bereitzustellen. Eine Fläche 12a der COC-Lage 12 bildet eine freiliegende Außenfläche des Folienverbunds 10. Die COC-Lage 12 weist bevorzugt eine Dicke von 9,8 bis 10,0 µm auf.

Mit der COC-Lage 12 durch Coextrusion verbunden ist eine Polyethylenlage 14. Die Polyethylenlage 14 ist vorzugsweise frei von COC und umfasst wenigstens 50 Gew.-% Metallocen-LLDPE C8. Im dargestellten Beispiel beträgt der Anteil an Metallocen-LLDPE C8 an der gesamten Polyethylenmasse der Polyethylenlage 14 mehr als 95 Gew.-%. Hierdurch wird eine extrem gute Haftung der COC-Lage 12 an der Polyethylen-Lage 14 und somit ein hoher Schutz vor Delamination erreicht. Die Verwendung von Metallocen-LLDPE des Typs C8 - im Gegensatz zu den grundsätzlich technisch ebenfalls möglichen Typen C4 oder C6 - führt bei gleichzeitig hoher Haftung der COC-Lage 12 zu einer Polyethylenlage 14 mit vorteilhaften Steifigkeitseigenschaften. Der Schmelzflussindex MFR der Polyethylenlage 14 beträgt etwa 8 g/10 min., gemessen gemäß ISO 1133 bei einer Prüftemperatur von 190 °C mit einem Prüfgewicht von 2,16 kg. Die Dichte der Polyethylenlage 14 beträgt bevorzugt 0,919 g/m².

Durch den gewählten Wert des Schmelzflussindex kann die Polyethylenlage 14, obwohl sie nicht außenliegende Lage des Folienverbunds 10 ist, an einer Siegelverbindung des Folienverbunds 10 auf der Seite der COC-Lage 12 teilnehmen. Hierdurch werden hohe Siegelfestigkeiten und eine sehr gute Qualität der Siegelnaht erreicht. Die COC-Lage 12 ist nicht nur Barrierelage des Folienverbunds 10, sondern auch dessen siegelfähige Lage.

Die Dicke der Polyethylenlage 14 beträgt im dargestellten Beispiel zwischen 10,8 und 11,0 µm. Somit sind die Polyethylenlage 14 und die COC-Lage 12 etwa gleich dick, wobei die COC-Lage 12 geringfügig dünner sein kann. Auch dieses ausgewogene Dickenverhältnis zwischen Polyethylenlage 14 und COC-Lage 12 trägt zur Erzielung dauerhaft fester und diffusionsdichter Siegelverbindungen bei.

Auf der von der COC-Lage 12 abgewandten Seite ist die Polyethylenlage 14 mit einer Verbindungslage 16 eines Ethylen-Acrylsäure-Copolymers (EAA) verbunden, um die COC-Lage 12 und die Polyethylenlage 14 mittels der Verbindungslage 16 mit einer Aluminiumlage 18 zu verbinden.

Die im dargestellten Beispiel zwischen 10,6 und 10,8 µm dicke Verbindungslage 16 wurde gemeinsam mit der Polyethylenlage 14 und der COC-Lage 12 coextrudiert und im Wege der Coextrusion auf die Aluminiumlage 18 aufgetragen.

Die als weitere Barrierelage dienende Aluminiumlage 18 ist im dargestellten Beispiel 8 bis 10 µm, vorzugsweise 9 µm dick.

Bereits ein Folienverbund aus den Lagen 12, 14, 16 und 18 ist ein stabiler Verbund, welcher zur Herstellung einer Verpackung für Produkte hervorragend geeignet ist, die pharmazeutische Wirkstoffe enthalten, wie Nikotin, Fentanyl oder Lidocain.

Um den genannten Verbund aus den Lagen 12, 14, 16 und 18 gegen ein unerwünschtes Öffnen bzw. Zerreißen durch neugierige Kinder zu schützen, die so an einen für Kinder gänzlich ungeeigneten verpackten pharmazeutischen Wirkstoff gelangen könnten, ist mittels einer Lage 20 aus lösungsmittelhaltigem Kaschierkleber mit einem Auftragsgewicht von 3 g/m² eine Lage 22 aus biaxial orientiertem Kunststoff, vorzugsweise aus Polyethylenterephthalat oder Polyamid, auf die von der COC-Lage 12 abgewandte Seite der Aluminiumlage 18 aufkaschiert.

Die Lage 22 kann an ihrer von der Aluminiumlage 18 wegweisenden Seite bedruckt sein, um einem Verbraucher Produktinformationen über ein mit dem Folienverbund 10 verpacktes Produkt mitzuteilen. Dann kann die Lage 22 eine der COC-Lage 12 entgegengesetzte außen liegende Lage des Folienverbunds 10 sein.

Anstelle einer Kunststofflage 22 kann die Lage 22 auch eine Papierlage sein.

Im dargestellten Beispiel von Figur 1 weist der Folienverbund 10 zusätzlich zur Kunststofflage 22, verbunden mittels einer weiteren Kaschierkleberlage 24 aus lösungsmittelhaltigem Kaschierkleber, eine außen liegende Papierlage 26 auf.

Die Papierlage 26 kann auf ihrer nach außen weisenden freiliegenden Fläche 26a bedruckt sein. Die Bedruckung kann durch eine Schutzschicht, etwa Klarlack, abgedeckt sein.

Im dargestellten Beispiel weist die Kunststofflage 22 eine Dicke von etwa 22,7 bis 23,3 µm auf. Die Papierlage 26 weist dagegen eine Dicke von etwa 39,5 bis 40,5 µm bei einem Flächengewicht von etwa 40 g/m² auf.

Die Gesamtdicke des Folienverbunds 10 beträgt somit zwischen 108,4 µm und 110,6 µm.

Der Folienverbund 10 weist nicht nur hervorragende Barriereeigenschaften gegen eine Migration und Diffusion von pharmazeutischen Wirkstoffen oder ihren Bestandteilen von der Außenfläche 12a der COC-Lage 12 zur Außenfläche 26a der Papierlage 26 auf. Der Folienverbund 10 hat überdies eine zur Bildung einer Verpackung sehr gut geeignete Steifigkeit und hervorragende Laufeigenschaften auf Verarbeitungsmaschinen zur Verarbeitung des Folienverbunds 10.

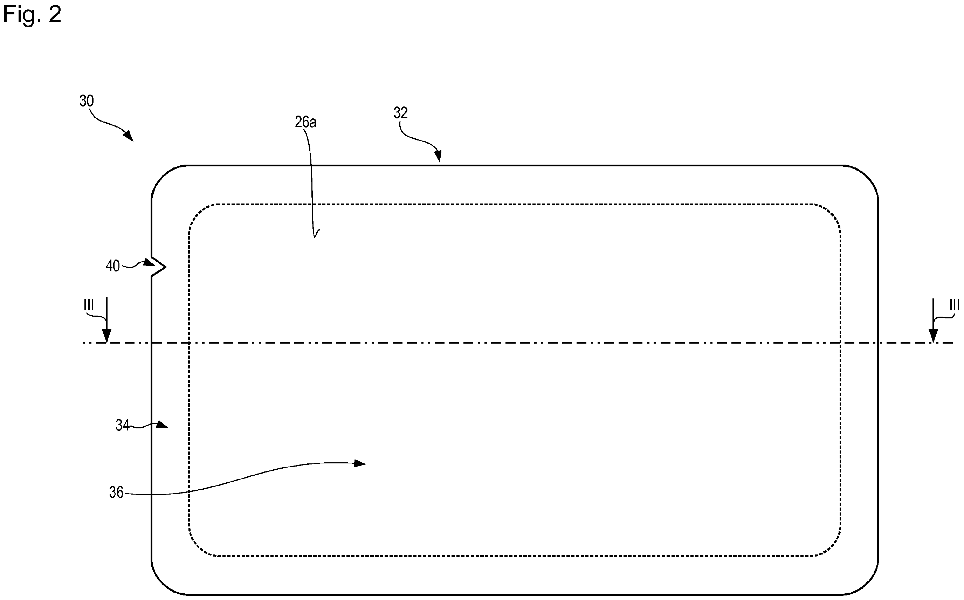

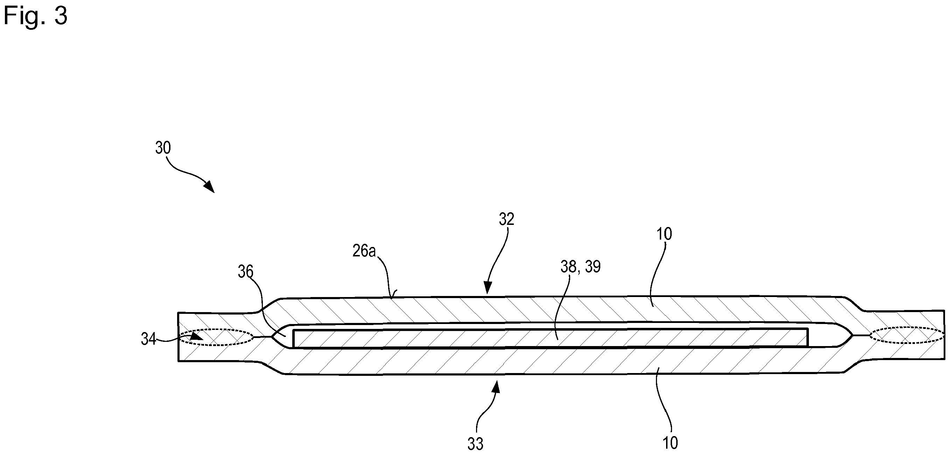

In Figur 2 ist eine aus zwei Folienverbundflecken des Folienverbunds 10 von Figur 1 gebildete Verpackung 30 zur Verpackung von flächigen Trägern von pharmazeutischen Wirkstoffen in Draufsicht gezeigt. Wegen der Draufsicht ist nur ein Folienverbundfleck 32 erkennbar. Ein weiterer Folienverbundfleck 33 befindet sich hinter dem in Figur 2 dargestellten Folienverbundfleck 32 und wird durch diesen verdeckt (siehe Figur 3). Man blickt in Figur 2 auf die Außenseite 26a der Papierlage 26 des Folienverbundflecks 32. Als "Folienverbundfleck" im Sinne der vorliegenden Anmeldung gilt jeder vorbestimmte Zuschnitt aus einem Folienverbund, unabhängig von der Art des Zuschnitts. Der Zuschnitt kann daher auch gestanzt oder dergleichen sein.

Die Verpackung 30 ist in Gestalt eines Vier-Rand-Siegelbeutels gebildet, mit einer umlaufenden Siegelnaht 34. Radial innerhalb der Siegelnaht 34 befindet sich ein Träger-Aufnahmeraum 36, in welchem ein flächiger Träger 38 eines pharmazeutischen Werkstoffs (siehe Figur 3) aufgenommen ist.

Als Aufreißhilfe kann die Verpackung 30 an wenigstens einer Stelle, etwa in einem Randbereich, eine Kerbe 40 aufweisen.

Figur 3 zeigt einen Längsschnitt durch die Verpackung 30 von Figur 2 längs der Schnittebene III-III von Figur 2. Die Schnittebene III-III ist orthogonal zur Zeichenebene von Figur 2 orientiert.

In Figur 3 ist im Längsschnitt durch die Folienverbundflecke 32 und 33 die Lagenverteilung innerhalb der Flecke 32 und 33 nicht mehr eigens dargestellt. Sie entspricht der in Figur 1 dargestellten Lagenverteilung des Folienverbunds 10. Die beiden COC-Lagen 12 der jeweiligen Folienverbundflecke 32 und 33 sind aufeinander zuweisend angeordnet und längs der Siegelnaht 34 miteinander verschweißt. In dem radial innerhalb der Siegelnaht 34 gebildeten Träger-Aufnahmeraum 36 ist ein transdermales Pflaster 39 als flächiger Träger 38 eines pharmazeutischen Wirkstoffs aufgenommen. Der Träger-Aufnahmeraum 36 ist somit durch die Siegelnaht 34 nach radial außen begrenzt und orthogonal hierzu durch die beiden Folienverbundflecke 32 und 33.

Die Siegelnaht 34 erstreckt sich in Dickenrichtung der jeweiligen Folienverbundflecke 32 und 33 nicht nur über die auch als Siegellage dienenden jeweiligen COC-Lagen 12, sondern über diese COC-Lagen 12 hinaus auch in die Polyethylenlagen 14 hinein. Die Siegelnaht 34 wird somit gebildet durch die sich vor der Siegelung berührenden COC-Lagen 12 der beiden Folienverbundflecke 32 und 33 und durch Material der aus Sicht der Berührstelle der beiden COC-Lagen 12 vor Herstellung des Siegelnaht 34 hinter den jeweiligen COC-Lagen 12 gelegenen Polyethylenlagen 14. Da es sich bei dem Material der COC-Lagen 12 ebenso wie bei dem Material der Polyethylenlagen 14 um ein Olefin handelt, sind die Materialien der beiden Lagen 12 und 14 miteinander kompatibel und können sich in der Siegelnaht 34 mischen.

## Patentansprüche

1. Folienverbund (10) zur Herstellung einer Verpackung (30) für flächige Träger (38) von pharmazeutischen Wirkstoffen, umfassend in Richtung von der Außenseite (26a) des Folienverbunds (10) zu seiner Innenseite (12a) eine Metalllage (18), eine Acrylsäure-haltige Verbindungslage (16), eine Polyethylenlage (14) und eine COC-Lage (12) aus Cycloolefin-Copolymer, wobei eine von der Metalllage (18) abgewandte Seite (12a) der COC-Lage (12) eine freiliegende Fläche des Folienverbunds (10) ist,
**dadurch gekennzeichnet, dass** die Polyethylenlage (14) Metallocen-Polyethylen C8 umfasst.

2. Folienverbund (10) nach Anspruch 1,
**dadurch gekennzeichnet, dass** der Anteil an Metallocen-Polyethylen am gesamten Polyethylen der Polyethylenlage (14) wenigstens 50 Gew.-% umfasst.

3. Folienverbund (10) nach Anspruch 1 oder 2,
**dadurch gekennzeichnet, dass** die Polyethylenlage (14) ein Metallocen-LLDPE umfasst.

4. Folienverbund (10) nach einem der vorhergehenden Ansprüche, **dadurch gekennzeichnet, dass** die Polyethylenlage (14) als Metallocen-Polyethylen nur ein Metallocen-Polyethylen C8 umfasst.

5. Folienverbund (10) nach einem der vorhergehenden Ansprüche,
**dadurch gekennzeichnet, dass** die Acrylsäure-haltige Verbindungslage (16) ein Ethylen-Acrylsäure-Copolymer umfasst oder aus diesem besteht.

6. Folienverbund (10) nach einem der vorhergehenden Ansprüche,
**dadurch gekennzeichnet, dass** jede der Lagen (16, 14, 12) aus Acrylsäure-haltiger Verbindungslage (16), Polyethylenlage (14) und COC-Lage (12) eine Lagendicke im Bereich von etwa 7 µm bis 15 µm, vorzugsweise im Bereich von 9 µm bis 12 µm aufweist, wobei sich bevorzugt die Dickenwerte der genannten Lagen (16, 14, 12) - bezogen auf die dünnste (12) der genannten Lagen (16, 14, 12) - um nicht mehr als 15 %, vorzugsweise um nicht mehr als 11 % unterscheiden.

7. Folienverbund (10) nach einem der vorhergehenden Ansprüche,
**dadurch gekennzeichnet, dass** er auf der von der COC-Lage (12) abgewandten Seite der Metalllage (18) wenigstens eine weitere Lage (22, 26) aus Papier oder/und Kunststoff aufweist.

8. Folienverbund (10) nach Anspruch 7,
**dadurch gekennzeichnet, dass** er auf der von der COC-Lage (12) abgewandten Seite der Metalllage (18) sowohl eine weitere Lage (26) aus Papier als auch eine weitere Lage (22) aus Kunststoff aufweist, wobei bevorzugt die Kunststofflage (22) näher bei der Metalllage (18) gelegen ist als die Papierlage (26).

9. Folienverbund (10) nach Anspruch 7 oder 8,
**dadurch gekennzeichnet, dass** die weitere Lage (22) aus Kunststoff ein biaxial orientierter Kunststoff ist.

10. Folienverbund (10) nach einem der Ansprüche 7 bis 9,
**dadurch gekennzeichnet, dass** die weitere Lage (22) aus Kunststoff Polyethylenterephthalat oder/und Polyamid ist.

11. Folienverbund (10) nach einem der Ansprüche 7 bis 10,
**dadurch gekennzeichnet, dass** die weitere Lage (26) aus Papier eine Dicke von 35 bis 45 µm, insbesondere von 39 bis 41 µm aufweist, oder/und ein Flächengewicht von 35 bis 45 g/m², insbesondere von 39 bis 41 g/m² aufweist, oder/und
dass die weitere Lage (22) aus Kunststoff eine Dicke von 19 bis 27 µm, insbesondere von 22 bis 24 µm aufweist oder/und ein Flächengewicht von 28 bis 36 g/m², insbesondere von 31 bis 33 g/m² aufweist.

12. Verpackung (30) für einen flächigen Träger (38) von pharmazeutischen Wirkstoffen, wie beispielsweise ein transdermales Pflaster (39), umfassend zwei Folienverbundflecke (32, 33) aus je einem Folienverbund (10) gemäß einem der vorhergehenden Ansprüche, welche unter Bildung eines Träger-Aufnahmeraums (36) mit aufeinander zuweisenden COC-Lagen (12) angeordnet und durch abschnittsweises Siegeln von COC-Lagenbereichen miteinander verbunden sind.

13. Verpackung (30) nach Anspruch 12,
**dadurch gekennzeichnet, dass** sie ein Vier-Rand-Siegelbeutel mit um den Träger-Aufnahmeraum (36) umlaufender Siegelnaht (34) ist.

14. Verpackung (30) nach Anspruch 12 oder 13 mit einem im Träger-Aufnahmeraum (36) aufgenommenen flächigen Wirkstoffträger (38), insbesondere transdermalem Pflaster (39), wobei der pharmazeutische Wirkstoff Nikotin oder/und Fentanyl oder/und Lidocain enthält.

## Claims

1. A film composite (10) for manufacturing a package (30) for planar carriers (38) of pharmaceutical active substances, encompassing, in a direction from the outer side (26a) of the film composite (10) toward its inner side (12a): a metal layer (18); an acrylic-acid-containing joining layer (16); a polyethylene layer (14); and a COC layer (12) made of cycloolefin copolymer, a side (12a) of the COC layer (12) which faces away from the metal layer (18) being an exposed surface of the film composite (10),
**characterized in that** the polyethylene layer (14) encompasses metallocene polyethylene C8.

2. The film composite (10) according to Claim 1,
**characterized in that** the proportion of metallocene polyethylene in terms of the total polyethylene of the polyethylene layer (14) is at least 50 wt%.

3. The film composite (10) according to Claim 1 or 2,
**characterized in that** the polyethylene layer (14) encompasses a metallocene LLDPE.

4. The film composite (10) according to one of the preceding claims, **characterized in that** the polyethylene layer (14) encompasses only a C8 metallocene polyethylene as a metallocene polyethylene.

5. The film composite (10) according to one of the preceding claims, **characterized in that** the acrylic-acid-containing joining layer (16) encompasses or is made of an ethylene-acrylic acid (EAA) copolymer.

6. The film composite (10) according to one of the preceding claims, **characterized in that** each of the layers (16, 14, 12), from among the acrylic-acid-containing joining layer (16), polyethylene layer (14), and COC layer (12), has a layer thickness in the range from approximately 7 µm to 15 µm, preferably in the range from 9 µm to 12 µm, the thickness values of the aforesaid layers (16, 14, 12), referred to the thinnest (12) of the aforesaid layers (16, 14, 12), differing by no more than 15%, preferably by no more than 11%.

7. The film composite (10) according to one of the preceding claims, **characterized in that** it comprises, on that side of the metal layer (18) which faces away from the COC layer (12), at least one further layer (22, 26) made of paper and/or plastic.

8. The film composite (10) according to Claim 7,
**characterized in that** it comprises, on that side of the metal layer (18) which faces away from the COC layer (12), both a further layer (26) made of paper and a further layer (22) made of plastic, the plastic layer (22) preferably being located closer to the metal layer (18) than is the paper layer (26).

9. The film composite (10) according to Claim 7 or 8,
**characterized in that** the further layer (22) made of plastic is a biaxially oriented plastic.

10. The film composite (10) according to one of Claims 7 to 9,
**characterized in that** the further layer (22) made of plastic is polyethylene terephthalate and/or polyamide.

11. The film composite (10) according to one of Claims 7 to 10,
**characterized in that** the further layer (26) made of paper has a thickness of 35 to 45 µm, in particular 39 to 41 µm, and/or a weight per unit area of 35 to 45 g/m², in particular 39 to 41 g/m²; and/or
the further layer (22) made of plastic has a thickness of 19 to 27 µm, in particular 22 to 24 µm, and/or a weight per unit area of 28 to 36 g/m², in particular 31 to 33 g/m².

12. A package (30) for a planar carrier (38) of pharmaceutical active substances, for example a transdermal patch (39), encompassing two film-composite sheets (32, 33), each made of a film composite (10) in accordance with one of the preceding claims, which are arranged with mutually facing COC layers (12) to form a carrier receiving space (36) and are joined to one another by local sealing of COC layer regions.

13. The package (30) according to Claim 12,
**characterized in that** it is a four-edge-sealed pouch having a sealed seam (34) surrounding the carrier receiving space (36).

14. The package (30) according to Claim 12 or 13, having a planar active-substance carrier (38), in particular a transdermal patch (39), received in the carrier receiving space (36), the pharmaceutical active substance containing nicotine and/or fentanyl and/or lidocaine.

## Revendications

1. Composite de film (10) pour la fabrication d'un emballage (30) pour des supports plats (38) de substances actives pharmaceutiques, comprenant, dans la direction allant du côté extérieur (26a) du composite de film (10) vers son côté intérieur (12a), une couche métallique (18), une couche de liaison contenant de l'acide acrylique (16), une couche de polyéthylène (14) et une couche de COC (12) en copolymère de cyclooléfine, une face (12a) de la couche de COC (12) opposée à la couche métallique (18) étant une face exposée du composite de film (10),
**caractérisé en ce que** la couche de polyéthylène (14) comprend du polyéthylène métallocène C8.

2. Composite de film (10) selon la revendication 1,
**caractérisé en ce que** la proportion de polyéthylène métallocène par rapport au polyéthylène total de la couche de polyéthylène (14) comprend au moins 50 % en poids.

3. Composite de film (10) selon la revendication 1 ou 2,
**caractérisé en ce que** la couche de polyéthylène (14) comprend un LLDPE métallocène.

4. Composite de film (10) selon l'une des revendications précédentes, **caractérisé en ce que** la couche de polyéthylène (14) comprend comme polyéthylène métallocène uniquement un polyéthylène métallocène C8.

5. Composite de film (10) selon l'une des revendications précédentes, **caractérisé en ce que** la couche de liaison contenant de l'acide acrylique (16) comprend ou est constituée d'un copolymère éthylène-acide acrylique.

6. Composite de film (10) selon l'une quelconque des revendications précédentes,
**caractérisé en ce que** chacune des couches (16, 14, 12) de la couche de liaison contenant de l'acide acrylique (16), de la couche de polyéthylène (14) et de la couche de COC (12) présente une épaisseur de couche dans la plage d'environ 7 µm à 15 µm, de préférence dans la plage de 9 µm à 12 µm, de préférence, les valeurs d'épaisseur desdites couches (16, 14, 12) - par rapport à la plus mince (12) desdites couches (16, 14, 12) - ne diffèrent pas de plus de 15 %, de préférence de plus de 11 %.

7. Composite de film (10) selon l'une quelconque des revendications précédentes,
**caractérisé en ce qu'**il présente, sur la face de la couche métallique (18) opposée à la couche COC (12), au moins une autre couche (22, 26) en papier ou/et en matière plastique.

8. Composite de film (10) selon la revendication 7,
**caractérisé en ce qu'**il présente, sur la face de la couche métallique (18) opposée à la couche COC (12), aussi bien une autre couche (26) en papier qu'une autre couche (22) en matière plastique, la couche en matière plastique (22) étant de préférence située plus près de la couche métallique (18) que la couche en papier (26).

9. Composite de film (10) selon la revendication 7 ou 8,
**caractérisé en ce que** la couche supplémentaire (22) en matière plastique est une matière plastique orientée biaxialement.

10. Composite de film (10) selon l'une des revendications 7 à 9,
**caractérisé en ce que** la couche supplémentaire (22) en matière plastique est du polyéthylène téréphtalate ou/et du polyamide.

11. Composite de film (10) selon l'une des revendications 7 à 10,
**caractérisé en ce que** la couche supplémentaire (26) en papier présente une épaisseur de 35 à 45 µm, notamment de 39 à 41 µm, ou/et un poids superficiel de 35 à 45 g/m², notamment de 39 à 41 g/m²,
ou/et
que la couche supplémentaire (22) en matière plastique présente une épaisseur de 19 à 27 µm, notamment de 22 à 24 µm, ou/et un poids superficiel de 28 à 36 g/m², notamment de 31 à 33 g/m².

12. Emballage (30) pour un support plat (38) de substances actives pharmaceutiques, comme par exemple un patch transdermique (39), comprenant deux feuilles de film composite (32, 33) constituées chacune d'un composite de film (10) selon l'une des revendications précédentes, qui sont disposées en formant un espace de réception de support (36) avec des couches de COC (12) se faisant face et qui sont reliées entre elles par un scellage par sections de zones de couches de COC.

13. Emballage (30) selon la revendication 12,
**caractérisé en ce qu'**il s'agit d'un sachet scellé à quatre bords avec un cordon de scellage (34) entourant l'espace de réception du support (36).

14. Emballage (30) selon la revendication 12 ou 13 avec un support plat de substance active (38), en particulier un patch transdermique (39), logé dans l'espace de réception du support (36), la substance active pharmaceutique contenant de la nicotine ou/et du fentanyl ou/et de la lidocaïne.
